(19)

**Europäisches Patentamt**

**European Patent Office**

**Office européen des brevets**

(11) **EP 3 517 934 A1**

(12) **EUROPEAN PATENT APPLICATION**

(43) Date of publication:
**31.07.2019 Bulletin 2019/31**

(21) Application number: **18153172.4**

(22) Date of filing: **24.01.2018**

(51) Int Cl.:
**G01N 21/21** (2006.01)  **G01N 21/31** (2006.01)
**G01N 21/41** (2006.01)  **A61B 5/00** (2006.01)
**G01N 33/483** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(71) Applicant: **I Love My Body Research S.r.l.**
**20124 Milano (IT)**

(72) Inventors:
• **Santucci, Giorgio**
  **20121 Milano (IT)**
• **Dobetti, Luca**
  **34151 Trieste (IT)**

(74) Representative: **Perani & Partners S.p.A.**
**Piazza Armando Diaz, 7**
**20123 Milano (IT)**

(54) **METHOD FOR THE DETERMINATION OF ANALYTES IN THE HAIR**

(57) The present invention relates to a method to determine the chemical composition of the hair, with special reference to possible analytes of interest like metals, vitamins, drugs, etc. The method for the determination of analytes in a hair, comprising the step of providing an hair sample and a plurality of pure analytes. The method is characterized in that it is based on processing light patterns, obtained from a spectrometer, in refracted polarized monochromatic light of said hair sample and said plurality of pure analytes, and in that the amount of each analyte present in the hair sample is calculated via unmixing.

EP 3 517 934 A1

**Description**

*Field of the invention*

The present invention relates to a method for the determination of analytes in the hair, according to the preamble of claim 1.

Particularly, the method is based on the interaction of polarized light with analytes present in a hair sample.

*Background of invention*

Hair analysis has been used from many years to assess human systemic level of metal elements. Hair is widely accepted for assessing toxic elements exposure and level of essential metals, despite the correlation with diseases, metabolic disorders and nutritional status is still object of extensive investigations.

Compared to other types of clinical specimens, hair has different use and even advantages over blood, urine and saliva. While the latter fluids tend to show current or recent body status, hair represent a longer timeframe strictly related to the long term metabolism (Watts D., Trace Elements and Other Essential Nutrients: Clinical application of tissue mineral analysis, InterClinical Laboratories Educational Publications, 2003; Klevay L.M. et al., Am. J. Clin. Nutr., 46, 233-236, 1987).

Hair mineral analysis is routinely carried out by means of a tissue mineral analysis, or thermo-mineralogram analysis (TMA).

The sampled hair is obtained by cutting the first 2-3 centimeters of growth closest to the scalp at the occiput (the nape of the neck) or latero-parietal area (the temples and the back of the head). The sample is washed and then mineralized to completely destroy the organic matrix while conserving the mineral content of the hair by means of an oxidizing acid solution. The aqueous acid solution obtained from mineralization is analyzed using a spectrophotometer in plasma emission at a temperature ranging from 8.000 to 10.000°C.

However, independent investigations have proven that TMA is not a reliable test by evaluating intra and inter-laboratory results (Schamberger R.J., Biological Trace Element Research, 87, 1-28, 2002; Drasch G. and Roider G., Journal of Trace Elements in Medicine and Biology, 16, 27-31, 2002; Hamilton T. and Schweinsberg F., Versicherungsmedizin, 56, 136-140, 2004; Seidel S. et al., JAMA, 285, 67-72, 2001).

TMA is generally not usable for individual diagnostic with few exceptions because of the large number of factors of individual and environmental influences and sources of error of the method of analysis.

Moreover, TMA cannot detect organic thermo-volatile or thermo-labile compounds, such as amino-acids, vitamins, hormones, etc., which are present in the hair and have an essential role in the metabolisms and nutritionals.

Alternative methods have been proposed to overcome the limitations given by TMA. US 7,688,943 teaches a method to determine the elemental concentrations in head and body hair by x-ray fluorescence analysis using synchrotron radiation. The metal (calcium) concentration is evaluated by the relative peak heights from fluorescent spectra of the element and background. This method is useful for cancer detection and protection as well as for diagnosing the calcium metabolism.

KR100372526 deals with a method for simultaneous analysis of multiple elements of hair sample by inductively coupled plasma mass spectrometry. The method includes the steps of washing a hair sample with surfactant, deionized water, and acetone, preparing a sample solution by radiating microwave to a nitric acid diluted solution receiving the washed hair sample, grouping the sample solution for detecting element by diluting the sample solution into two groups of 200-300 times (part per billion level) and 2000-30000 times (part per million level), and simultaneously analyzing the multiple elements contained in the hair sample by carrying out the inductively coupled plasma mass spectrometry for the diluted sample solutions.

US 7,625,708 and US 7,629,129 provide a method of using hair follicle bulbs as bio-dosimeters for the detection of chemical exposure. The methods utilize intact, plucked hair follicle bulbs to monitor real-time or near real-time changes in the levels of specific follicular bulb biomarkers to determine exposure to toxicants. By utilizing the living, responsive cells in the plucked hair follicle bulb in an immunohistochemical analysis, the various embodiments mitigate the risks of false positives associated with segmental hair analysis and avoid the more invasive collection required for serum and urine analysis.

US20100002224 describes a device for remotely sensing the presence of hard keratin by means of near and short wavelength infrared light and a microprocessor with algorithms to determine the presence of hard keratin based upon the identification of its spectral characteristic.

Polarized light is used in hair analysis for determine the morphology and, thus, the physiological conditions of the hair.

US20100105102 describes a method and device to analyze an electronic image of skin or hair samples. The invention relates in general to the field of surface analysis, for identifying biological markers to analyze skin and hair by means of a mixture of agents which are responsive to light (phosphorescence), pressure or change color (chemiluminescent) by wetting, pH or chemical reaction and thus provide measurement of transmitted and reflected light.

EP0440907 proposes an apparatus for carrying out hair morphological analyses, in which optical and chemico-physical characteristic data of the hair structure and hair care measures carried out are stored, evaluated and displayed by means of a computer.

EP1636752 describes a system for producing images having mixed colors and analyzing the images to provide deconvoluted images in which colors (in general two) are unmixed from photographic pixels. One color may be used simply for improved visibility of all tissue in the slide, while the other color may bind to protein receptor in tissue affected by a particular disease and provides color data of diagnostic significance, commonly immunohistochemical. Three spectral channels (wavelengths) are used to define the reference pattern of the two end members (dyes), whose full spectral patterns in ultraviolet-visible range is well known.

With the same principle, WO2008025006 describes systems and methods for spectral unmixing of in vivo light data (from mouse). The spectral unmixing separates image data according to spectra from multiple internal light sources in an effort to isolate one or more spectrum of interest. The spectral unmixing obtains images with a combination of different and known excitation and emission limits. The spectral unmixing then uses an iterative solution process to separate spectra for the multiple fluorescent light sources, and provides a spectrum and/or a spatial distribution map, composed by a limited number of spectral wavelengths, for at least one of the internal light sources.

Both the above applications utilize the transmitted intensity of a limited number of selected wavelengths in UV-visible spectrum of non polarized light.

EP1636752 and WO2008025006 describe the determination of oligo-elements in hair (a use not disclosed in these documents), was found unsatisfactory in our experiments: the relevant tests, using a number of discrete channels (wavelengths) for the quantitative determination of each element, yielded results partially inconsistent with what expected the physiological state of the patient according to the medical literature, and a relatively high mean square error arose from the application of the unmixing methodology; moreover, as the number of the compounds analyzed at the same time increased, some of them could not be detected (values equal to zero); furthermore, non-polarized UV-Visible spectral patterns could only be obtained for some organic substances, while no absorption occurred for other organic substances and for pure mineral elements.

EP2518474 describes a method to determine the chemical composition of the hair by irradiating the hair with polarized light, obtaining a microscope digital pixel-based image (pattern) in refracted polarized light (remote sensing), and elaborating the pattern according to a deconvolution or unmixing method, wherein the amount of each analyte present in the hair sample is calculated. The method allows the determination of a large number of analytes in the hair, such as metals, aminoacids, drugs, hormones, vitamins, pollutants, poisons, etc., thus providing useful information of the physiological and pathological status of the patient beside the standard diagnostics such as blood and urine. The method of EP2518474 requires to irradiate with polarized light (white light) a number of pure optically active analytes (i.e. those possibly present in the hair) and to construct for each of them a light pattern of double refracted light in a way below described; then the hair to be analyzed is irradiated in the same way, and its light pattern is constructed accordingly. The constructed light patterns are then compared against each other via a computer-assisted unmixing processing, allowing to determine the relative contribution of each analyte present in the hair sample. Once the relative contribution of the analyte is determined, its absolute amount and/or concentration in the hair sample (ppm, $\mu$g/mg, etc.) is easily calculated in standard way.

The light patterns from refracted light are constructed from a microscope digital picture of the sample in question (pure analyte or hair to be analyzed) in polarized light; a matrix of points is then constructed from this picture, wherein each point corresponds to a single pixel of the picture, and is defined by the wavelength and absorbance value of said pixel; all pixels of the picture are included in the matrix; the matrix includes all possible wavelengths of the pixels, i.e. the sampling is not limited to discrete selected wavelengths.

*Problem of the known art*

The method of EP2518474while being advantageous is not exempt from problems.

In fact, the examples described in EP2518474 teach the collection of intensities coming from a discrete number of selected wavelengths (i.e. six wavelengths).

It results in a lower accuracy or higher mean square error of the analytical results.

Particularly, the method does not obtain a proper level of accuracy of the results due to the use of a white light and by the use of microscope digital picture.

SUMMARY OF THE INVENTION

It has been found that even more accurate results of the hair analysis are obtained by using a monochromatic source of emitted light, rather than sources that cover a wide range of emitted wavelengths, such as white light (normally used in microscopy) or LED light.

The principle is analogous to that in EP2518474 with the main difference in the type of light used to go through the pure analytes and the hair sample for collecting the matrix of points (raw data).

Particularly, single wavelengths are obtained by decomposition of white light by mean of an optical prism or spectrometer (i.e. a UV-VIS spectrometer) or by using multiple monochromatic laser sources. Then, for each wavelength a couple of data - wavelength and intensity at that wavelength - is collected to make the matrix of pure analytes (or end members) and hair samples.

The method comprises the step of irradiating with monochromatic polarized light a plurality of pure analytes (i.e. those possibly present in the hair) and to construct for each of them a light pattern. In particular, the light pattern consists in a matrix of points, each point defining the wavelength and absorbance of that wavelength. The hair to be analyzed is irradiated in the same way, and its light pattern is constructed accordingly.

The constructed light patterns are then compared against each other via a computer-assisted unmixing processing (also called deconvolution), allowing to determine the relative concentration (ppm, $\mu$g/mg, etc.) of each analyte present in the hair sample.

The light patterns of the sample in question (pure analyte or hair to be analyzed) in polarized light are constructed from a spectrometer. A matrix of points is then constructed, wherein each point corresponds to wavelength and absorbance/intensity value of that wavelength.

As a result of the above, very precise light patterns are obtained for the standard analytes and for the hair sample.

The matrix data are then processed via software-assisted unmixing; the use of the unmixing algorithm determines the relative concentration of each single analyte to the spectral patterns, providing a very precise and reproducible assessment of the analytes in the hair, including those being present in traces.

The method according to the invention, comprises the steps of:

a) [calibration phase]: constructing a light pattern for each pure analyte (end member);
b) [measuring phase]: constructing a light pattern of the hair sample under analysis, preferably in the bulb area;
c) [determination phase] comparing the patterns obtained in a) against the pattern obtained in b) and determining the relative concentration of each analyte in the hair sample, via software-assisted unmixing processing.

The calibration phase a) needs not be performed every time before analysing a hair sample. It is generally sufficient to perform it once and then use the resulting light patterns of the pure analytes (end members) as references for an unlimited number of hair samples analysis; these will then include steps b) and c) only.

The calibration and measuring phases a) and b) require the same methodology of:

- refracting an incident monochromatic polarized light beam through the analyte/hair sample;
- constructing a spectral pattern (matrix of points), each point being characterized by the wavelength and absorbance values at that wavelength.

In order to obtain the spectral pattern, the pure analytes (or hair samples) are placed in a spectrometer chamber, where the spectrometer, preferably provided with polarizing filters, can have a white light source with prism which decomposes the beam in monochromatic wavelengths or the monochromatic light source can be generated by laser light at different wavelengths (or in other way well-known by an expert).

The pure analyte is placed in the spectrophotometer chamber in liquid form or in tiny layer of 30 $\mu$m if in solid form. If the pure analyte cannot be analyzed for any reason (i.e. sodium), a chemically pure compound with highly similar characteristics can be used (i.e. oxide, carbonate).

The irradiation with monochromatic polarized light of the pure analytes/hair sample is performed by means of light sources which work preferably in the visible range typically at a wavelength from 350 to 800 nm.

Alternatively, the irradiation, described above, is performed by means of light sources which work in UV range with a wavelength from 10 to 400 nm.

A more extensive range of wavelengths can be used (180-950 nm) but the level of performances (sensitivity) is slightly decreases approaching the upper and lower wavelength limits.

In particular, the process of unmixing is an algorithm-based process used to reverse the effects of convolution on recorded data.

It shall be noted that the concept of deconvolution is widely used in the techniques of signal processing and image processing. These techniques are in turn widely used in many scientific and engineering disciplines.

In general, the object of deconvolution (unmixing) is to find the solution of a convolution equation of the form:

$$y(\lambda) = \sum_{k=1}^{m} a_k e_k(\lambda) + n(\lambda)$$

where $y(\lambda)$ represents the recorded signal and is a linear mixing of $e_k(\lambda)$, reference function of the k pure elements or end members, and $a_k$, relative positive weight of each pure elements, the sum of such relative positive weights being equal to one.

Unmixing is the determination of the $a_k$ weights which minimize the E function by means of the ordinary least square statistical method as

$$E = \sum_{i=1}^{c} \left| y(\lambda_i) - \sum_{k=1}^{m} \alpha_k e_k(\lambda_i) \right|^2$$

knowing the reference function of each pure element $e_k(\lambda)$.

When the reference function is a wavelength spectrum (spectral pattern), hyper-spectral data are used to determine what elements or materials are present in a scene by means of the unmixing method.

The state of the art also teaches that, when a high number of elements are detected and compose the unknown convoluted spectrum, only a relatively limited number of elements effectively have a positive $a_k$ weight and provide a contribution to the amplitude of a particular single $\lambda_k$ wavelength. In other words, given k the number of elements contributing to the convoluted spectrum to be detected, only m elements (with m<k) effectively provide a contribution to the amplitude of a particular single $\lambda_k$ wavelength.

The contribution of the other k-m elements to the amplitude of that particular $\lambda_k$ wavelength is practically zero.

Therefore, the quantitative determination of the n elements in a convoluted spectrum can be carried out by weighing the relative presence of $m_1$ elements in the $\lambda_1$ wavelength, $m_2$ elements in the $\lambda_2$ wavelength, $m_j$ elements in the $\lambda_j$ wavelength. Then, the relative presence of the n elements is averaged among the $\lambda_j$ wavelengths detected.

Well-known unmixing algorithm, generally described above, has been used in the *Interactive Data Language* (IDL) elaboration from the pictures. The IDL software evaluates the wavelength and absorbance in the hair sample and then compares the absorbance of all the pure analytes at that single wavelength value. A corresponding list of analytes in descending order of absorbance is produced accordingly; within this list, only a fraction of analytes, i.e. those with highest absorbance, will be considered for processing by unmixing. In the present invention, the threshold is preferably set at about 15% of the total number of analytes under analysis, since the contribution of absorbance from the other analytes is negligible or zero. Thus, for example, in a list of 70 analytes, only the first 10 will be considered for the unmixing processing.

Wishing to further increase the precision of the method, the same sample (pure analyte or hair) can be scanned *n* times (e.g. 2 to 15, preferably 4 to 12), producing *n* pre-matrixes, whose data are then averaged to construct the actual final matrix. In fact, the precision of the method increases by increases the number of the scans and consequent averaged till a plateau after that the precision remains unchanged. This plateau is usually reached after 4-5 runs, although a good precision is often reached after 2-3 run. Sometimes, further slight improvements of precision are obtained after 4-5 runs, which do not modify substantially the outcomes of the analysis.

In step c), the well-known unmixing algorithm is applied via a software-assisted processing, which enables reaching the final result in a reasonable amount of time. In the invention said unmixing is performed preferably with an adaptation, in that it considers the relative weight of only the most absorbing analytes at each detected wavelength. This correlation can be established by comparing the absorbance of all the pure analytes at a single wavelength value.

In the diagnostic test, each hair scan is analyzed (wavelength and absorbance). The IDL software applies the unmixing elaboration of the spectrum, by selecting among all the end-members (pure analytes) those effectively contributing to the absorbance at that wavelength. The automatic elaboration of hair spectrum will provide then the concentration of each end-members and the quali-quantitative composition of these analytes in the hair.

Although the unmixing processing provides data relevant to the concentration of all analytes sampled in the calibration phase, they need not be displayed as a whole to the user: in fact, depending on the type of analysis, it may be interesting to obtain information on only some analytes (e.g. vitamins, specific drugs, metals, etc.): in this case the software can be programmed to display information relevant to the analytes of interest.

The software may further be adapted, by standard procedures, to store and/or further elaborate the analyzed data, so as to build medical databases assisting the doctor in patient diagnosis, or for research purpose.

Wishing to increase the diagnostic relevance of the present method, the latter will be preferably repeated on different hair samples of the patient, preferably from different areas of the scalp, and averaging the results obtained: this will minimize the possible variability of composition among different hair of a patient.

The analytes considered in the present method are the pure elements and organic molecules, which are potentially present, permanently and/or temporarily in the hair. The pure elements must possess the capacity of refracting an incident polarized light beam, thus generating a colorful refraction spectrum.

The pure elements are minerals, also called metals, nutrient or toxic according to their biological function and definition, non-metal elements and, more in general, organic molecules.

Not limiting examples of organic molecules are amino-acids, vitamins, hormones, doping or psychotropic compounds, poisons, pollutants.

Not limiting examples of nutrient metals are silver, calcium, cobalt, chrome, iron, lithium, magnesium, manganese, molybdenum, cadmium, potassium, selenium, sodium, strontium, copper, tin, vanadium, zinc.

Not limiting examples of toxic metals are aluminum, arsenic, barium, cadmium, mercury, nickel, lead, uranium.

Not limiting examples of no metal elements are phosphorus, fluorine, iodine, silica, sulfur.

Not limiting examples of amino-acids are aspartic acid, glutamic acid, alanine, arginine, cysteine, phenylalanine, glycine, isoleucine, hystidine, leucine, lysine, methionine, proline, serine, taurine, tyrosine, threonine, tryptophan, valine.

Not limiting examples of vitamins are folic acid, niacin, vitamin A, vitamin B1, vitamin B2, vitamin B5 or pantothenic acid, vitamin B6, vitamin B12, vitamin C, vitamin D, vitamin E, vitamin H or biotin, vitamin K.

Not limiting examples of hormones are estrogen, progesterone, oxytocin, dopamine, serotonin, noradrenalin, adrenalin, testosterone.

Not limiting examples of doping and psychotropic drugs are lysergic acid, cocaine, heroin, morphine, opium extracts and derivatives, cannabis extracts and derivatives, temazepam, lorazepam, and, more in general, benzodiazepine drugs, synthetic drugs.

As documented in the experimental section, the method of the present invention allows to simultaneously detect a very large number of analytes in the hair, including those (cf. minerals) not detectable using the conventional light reflectance-based methods of the prior art; the method proved accurate and reproducible, involving a very low mean standard error; furthermore it proved capable to detect elements even in traces, not otherwise found by said prior art methods.

The invention is now described by reference to the following non-limiting examples.

Methods compared

The following methods are considered for the comparison:

a) Poured on a microscope glass and placed in a microscope equipped with polarizing filters. Eight digital pictures of refracted polarized light by the elements are made for each element using a digital camera with at least seven million pixels. The wavelengths of the refracted polarized light and the relative transmittance component are determined in each pixel and used to construct the spectral matrix reference patterns (end members) as a mean of the eight pictures.

b) Poured in a chamber of a spectrometer equipped with polarizing filters and monochromatic light beams, as described above. The spectral matrix reference patterns (end members) is constructed by taking a matrix of monochromatic wavelength and intensity at that wavelength.

A sample of 70 elements and compounds has been selected. They include:

- 31 pure elements (minerals), such as silver, calcium, cobalt, chrome, iron, lithium, magnesium, manganese, molybdenum, cadmium, potassium, selenium, sodium, strontium, copper, tin, vanadium, zinc, aluminum, arsenic, barium, cadmium, mercury, nickel, lead, uranium, phosphorus, fluorine, iodine, silica, and sulfur;
- 13 vitamins, such as folic acid, niacin, vitamin A, vitamin B1, vitamin B2, vitamin B5, vitamin B6, vitamin B12, vitamin C, vitamin D, vitamin E, vitamin H, and vitamin K;
- 19 amino-acids, such as aspartic acid, glutamic acid, alanine, arginine, cysteine, phenylalanine, glycine, isoleucine, hystidine, leucine, lysine, methionine, proline, serine, taurine, tyrosine, threonine, tryptophan, and valine;
- 7 hormones, such as estrogen, progesterone, oxytocin, dopamine, serotonin, noradrenalin, and testosterone.

The two methods are used to obtain a spectral pattern reference (end members) from the above elements and compounds for the unmixing algorithm-based process in the following examples.

Example 1

Five vitamins, vitamin A, vitamin B1, vitamin B2, vitamin C and vitamin D, are analyzed.

One single hair is then taken from the latero-parietal area of the head.

All the spectral patterns of the five vitamins are taken using method a.

All the spectral patterns of the five vitamins are taken using method b.

The relative amount of the five vitamins in the hair is then evaluated by means of the unmixing algorithm-based process (ordinary least square) having as spectral pattern references (end members) those obtained by a. or b. methods.

The results are reported in Table 1 and show comparable quantitative outcomes but a much higher statistical significance using method b.

*Table 1. Concentration of the five vitamins in the analyzed hair and mean square error (MSE) using method a. and b.*

| Vitamin | Concentration (a.) | Concentration (b.) |
|---|---|---|
| A | 300,02 ($\mu$g/g) | 306,26 ($\mu$g/g) |
| B1 | 8,16 ($\mu$g/g) | 8,75 ($\mu$g/g) |
| B2 | 9,96 ($\mu$g/g) | 9,20 ($\mu$g/g) |
| C | 10,99 ($\mu$g/g) | 11,58 ($\mu$g/g) |
| D | 42,10 ($\mu$g/g) | 39,98 ($\mu$g/g) |
| MSE | 18,888 | 9,216 |

Example 2

Six amino-acids, arginine (Arg), cysteine (Cys), glycine (Gly), leucine (Leu), proline (Pro) and valine (Val), are analyzed.

One single hair is then taken from the latero-parietal area of the head.

All the spectral patterns of the six amino-acids are taken using method a.

All the spectral patterns of the six amino-acids are taken using method b.

The relative amount of the six amino-acids in the hair is then evaluated by means of the unmixing algorithm-based process (ordinary least square) having as spectral pattern references (end members) those obtained by a. or b. methods.

The results are reported in Table 2 and show comparable quantitative outcomes but a much higher statistical significance using method b.

*Table 2. Concentration of the six amino-acids in the analyzed hair and mean square error (MSE) using method a. and b.*

| Amino-acid | Concentration (a.) | Concentration (b.) |
|---|---|---|
| Arg | 39,14 ($\mu$g/g) | 43,12 ($\mu$g/g) |
| Cys | 77,00 ($\mu$g/g) | 80,11 ($\mu$g/g) |
| Gly | 12,14 ($\mu$g/g) | 11,99 ($\mu$g/g) |
| Leu | 40,90 ($\mu$g/g) | 42,88 ($\mu$g/g) |
| Pro | 28,04 ($\mu$g/g) | 30,09 ($\mu$g/g) |
| Val | 58,57 ($\mu$g/g) | 60,03 ($\mu$g/g) |
| MSE | 34,998 | 15,928 |

Example 3

Three hormones, progesterone, oxytocin and testosterone, are analyzed.

One single hair is then taken from the latero-parietal area of the head.

All the spectral patterns of the three hormones are taken using method a.

All the spectral patterns of the three hormones are taken using method b.

The relative amount of the three hormones in the hair is then evaluated by means of the unmixing algorithm-based process (ordinary least square) having as spectral pattern references (end members) those obtained by a. or b. methods.

The results are reported in Table 3 and show comparable quantitative outcomes but a much higher statistical significance using method b.

*Table 3. Concentration of the three hormones in the analyzed hair and mean square error (MSE) using method a. and b.*

| Hormones | Concentration (a.) | Concentration (b.) |
|---|---|---|
| Progesterone<br>Oxytocin<br>Testosterone | 18,28 ($\mu$g/g)<br>500,11 ($\mu$g/g)<br>160,12 ($\mu$g/g) | 22,00 ($\mu$g/g)<br>489,99 ($\mu$g/g)<br>154,44 ($\mu$g/g) |
| MSE | 12,415 | 5,900 |

Example 4

All the elements and compounds in Example 1 are analyzed.

One single hair is then taken from the latero-parietal area of the head.

All the spectral patterns of the all elements and compounds are taken using method a.

All the spectral patterns of the all elements and compounds are taken using method b.

The relative amount of the all the elements and compounds in the hair is then evaluated by means of the unmixing algorithm-based process (ordinary least square) having as spectral pattern references (end members) those obtained by a. or b. methods. Mean square error (Table 4) shows a much higher statistical significance using method b.

*Table 4. Mean square error (MSE) using method a. and b.*

| Hormones | Concentration (a.) | Concentration (b.) |
|---|---|---|
| MSE | 317,009 | 145,096 |

Of course, those skilled in the art can bring numerous modifications to the variants described above, in order to satisfy contingent and specific requirements, all in any case covered by the scope of protection as defined by the following claims.

**Claims**

1. A method for the determination of analytes in a hair, comprising the step of providing an hair sample and a plurality of pure analytes, the method being **characterized in that** is based on processing light patterns, obtained from a spectrometer, in refracted polarized monochromatic light of said hair sample and said plurality of pure analytes, **and in that** the amount of each analyte present in the hair sample is calculated via unmixing.

2. The method of claim 1 comprising the steps of:

   a) a calibration phase, wherein the light patterns of pure analytes (end members) are constructed;
   b) a measuring phase, wherein the light pattern of the hair sample is constructed;
   c) a determination phase, wherein the light patterns of a) is compared against the light pattern of b) and the relative concentration of each analyte in the hair sample is determined via software-assisted unmixing processing.

3. The method of the claim 1, wherein each light pattern consists in a matrix of points, each point defining the monochromatic wavelength and absorbance of that wavelength.

4. The method according to anyone of claims 1-3, wherein the light patterns are constructed by considering all possible wavelengths, preferably in the range of the visible.

5. The method according to anyone of claims 2-4, wherein the light patterns of pure analytes (end members) are obtain at least one time.

6. The method according to anyone of claims 1-5, wherein said light patterns are obtained by averaging the data from 2-15 scans of the same sample.

7. The method according to anyone of claims 1-6, wherein the unmixing utilizes the relative weight of the most absorbing analytes at each detected wavelength.

8. The method of claim 7, wherein said most absorbing analytes corresponding to about 15% of the total number of analytes under testing.

9. The method according to claims 1-8, wherein the analytes include one or more among metals, non-metal elements, aminoacids, vitamins, hormones, doping and/or psychotropic drugs, poisons, pollutants.

10. The method according to claim 9, wherein:

   - the metals comprise one or more among: silver, calcium , cobalt, chrome, iron, lithium, magnesium, manganese, molybdenum, cadmium, potassium, selenium, sodium, strontium, copper, tin, vanadium, zinc, aluminum, arsenic, barium, cadmium, mercury, nickel, lead, uranium;
   - the non-metal elements comprise one or more among phosphorus, fluorine, iodine, silica, sulfur;
   - the amino-acids comprise one or more among aspartic acid, glutamic acid, alanine, arginine, cysteine, phenylalanine, glycine, isoleucine, hystidine, leucine, lysine, methionine, proline, serine, taurine, tyrosine, threonine, tryptophan, valine;
   - the vitamins comprise one or more among folic acid, niacin, vitamin A, vitamin B1, vitamin B2, vitamin B5 or pantothenic acid, vitamin B6, vitamin B12, vitamin C, vitamin D, vitamin E, vitamin H or biotin, vitamin K;
   - the hormones comprise one or more among estrogen, progesterone, oxytocin, dopamine, serotonin, noradrenalin, adrenalin, testosterone.

Europäisches
Patentamt

European
Patent Office

Office européen
des brevets

# EUROPEAN SEARCH REPORT

Application Number

EP 18 15 3172

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 2 518 474 A1 (WELL DYNAMICS APPLIC S R L WDA [IT]) 31 October 2012 (2012-10-31) * the whole document * | 1-10 | INV. G01N21/21 G01N21/31 G01N21/41 A61B5/00 G01N33/483 |
| A | WO 2012/164441 A1 (KONINKL PHILIPS ELECTRONICS NV [NL]; HEINRICH ADRIENNE [NL]; VAN HEESC) 6 December 2012 (2012-12-06) * abstract * | 1-10 | |

TECHNICAL FIELDS
SEARCHED      (IPC)

G01N
A61B

The present search report has been drawn up for all claims

| Place of search | Date of completion of the search | Examiner |
|---|---|---|
| Munich | 10 April 2018 | Ruchaud, Nicolas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 18 15 3172

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2018

| Patent document cited in search report | | | Publication date | Patent family member(s) | | Publication date |
|---|---|---|---|---|---|---|
| EP 2518474 | A1 | 31-10-2012 | | NONE | | |
| WO 2012164441 | A1 | 06-12-2012 | | BR 112013030487 | A2 | 27-09-2016 |
| | | | | CN 103620380 | A | 05-03-2014 |
| | | | | EP 2715316 | A1 | 09-04-2014 |
| | | | | JP 5965995 | B2 | 10-08-2016 |
| | | | | JP 2014519608 | A | 14-08-2014 |
| | | | | RU 2013158331 | A | 10-07-2015 |
| | | | | US 2014081148 | A1 | 20-03-2014 |
| | | | | WO 2012164441 | A1 | 06-12-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7688943 B
- KR 100372526
- US 7625708 B
- US 7629129 B
- US 20100002224 A

- US 20100105102 A
- EP 0440907 A
- EP 1636752 A
- WO 2008025006 A
- EP 2518474 A

**Non-patent literature cited in the description**

- **WATTS D.** Trace Elements and Other Essential Nutrients: Clinical application of tissue mineral analysis. *InterClinical Laboratories Educational Publications,* 2003
- **KLEVAY L.M. et al.** *Am. J. Clin. Nutr.,* 1987, vol. 46, 233-236

- **SCHAMBERGER R.J.** *Biological Trace Element Research,* 28 January 2002, vol. 87
- **DRASCH G. ; ROIDER G.** *Journal of Trace Elements in Medicine and Biology,* 2002, vol. 16, 27-31
- **HAMILTON T. ; SCHWEINSBERG F.** *Versicherungsmedizin,* 2004, vol. 56, 136-140
- **SEIDEL S. et al.** *JAMA,* 2001, vol. 285, 67-72